(19) Europäisches Patentamt
European Patent Office
Office européen des brevets

(11) Numéro de publication : **0 346 240 B1**

# (12) FASCICULE DE BREVET EUROPEEN

(45) Date de publication du fascicule du brevet : **02.06.93 Bulletin 93/22**

(51) Int. Cl.[5] : **C07D 279/28,** A61K 31/54

(21) Numéro de dépôt : **89401603.9**

(22) Date de dépôt : **09.06.89**

## (54) Nouveaux dérivés de la phénothiazine, leur préparation et les compositions qui les contiennent.

(30) Priorité : **10.06.88 FR 8807772**

(43) Date de publication de la demande : **13.12.89 Bulletin 89/50**

(45) Mention de la délivrance du brevet : **02.06.93 Bulletin 93/22**

(84) Etats contractants désignés : **AT BE CH DE ES FR GB GR IT LI LU NL SE**

(56) Documents cités : **US-A- 3 112 310**

(73) Titulaire : **RHONE-POULENC SANTE
20, avenue Raymond Aron
F-92160 Antony (FR)**

(72) Inventeur : **Garret, Claude
129 rue Jean Jaurès
F-94120 Fontenay sous Bois (FR)**
Inventeur : **Guyon, Claude
17 bis avenue Henri Martin
F-94100 Saint Maur des Fosses (FR)**
Inventeur : **Plau, Bernard
1 Impasse des Rouges-Gorges
F-94260 Frèsnes (FR)**
Inventeur : **Taurand, Gérard
2/124 allée Jean de la Bruyère
F-94000 Créteil (FR)**

(74) Mandataire : **Savina, Jacques et al
RHONE-POULENC SANTE, Service Brevets,
20 Avenue Raymond Aron
F-92165 Antony Cédex (FR)**

EP 0 346 240 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés de la phénothiazine de formule générale :

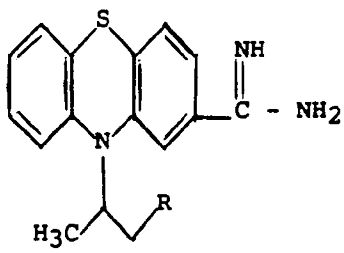

(I)

leurs sels d'addition avec les acides, leur préparation et les compositions pharmaceutiques qui les contiennent.

Dans le brevet US 3 112 310 ont été décrits des amides dérivés de la phénothiazine de formule générale :

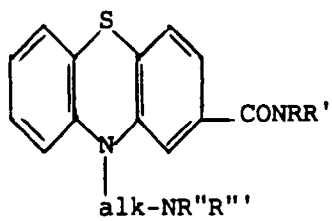

dans laquelle R et R' sont notamment des atomes d'hydrogène, pour leur activité sur le système nerveux central.

Dans le brevet belge 612 885 ont été décrits des thioamides dérivés de la phénothiazine de formule générale :

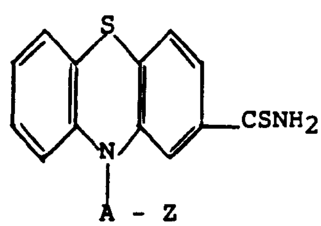

dans laquelle A est une chaîne carbonée et Z est notamment un radical dialcoylamino ou un hétérocycle azoté, actifs comme neuroleptiques, antiémétiques, adrénolytiques et anti-tuberculeux.

Il a été trouvé que les produits de formule générale (I) dans laquelle :

le symbole R représente

- soit un radical

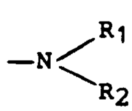

(IIa)

  pour lequel les symboles $R_1$ et $R_2$ identiques ou différents représentent des radicaux alcoyle, hydroxyalcoyle, acétyloxyalcoyle ou forment ensemble avec l'atome d'azote auquel ils sont rattachés, un hétérocycle contenant 4 à 7 chaînons, éventuellement substitué par 1 ou 2 radicaux alcoyle, hydroxyalcoyle ou acétyloxyalcoyle,
- soit représente un radical

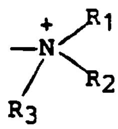

(IIb)

pour lequel $R_1$ et $R_2$ sont définis comme précédemment et $R_3$ représente un radical alcoyle ou phénylalcoyle, sous leurs formes isomères ou leurs mélanges, ainsi que leurs sels, qui manifestent une affinité pour les récepteurs opiacés de type Mu, présentent une activité antidiarrhéique sans pour autant entraîner d'effet secondaire au niveau du système nerveux central.

Dans la formule générale (I), il est entendu que les radicaux alcoyle sont droits ou ramifiés et contiennent 1 à 4 atomes de carbone.

Selon l'invention, les produits de formule générale (I) peuvent être obtenus par action de l'ammoniac sur un iminoéther de formule générale :

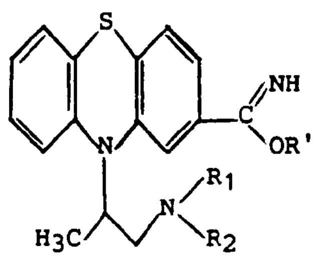

(III)

dans laquelle $R_1$ et $R_2$ sont définis comme précédemment et R' est un radical alcoyle contenant 1 à 10 atomes de carbone, suivie le cas échéant lorsque l'on veut obtenir un produit pour lequel R est un radical de formule générale (IIb) de l'action d'un produit de formule générale :

$$R_3 - X \qquad (IV)$$

dans laquelle $R_3$ est défini comme précédemment et X représente un atome d'halogène choisi parmi l'iode, le brome ou le chlore, ou un radical alcoylsulfonyloxy ou phénylsulfonyloxy dont le radical phényle est éventuellement substitué par un ou plusieurs atomes d'halogène ou radicaux alcoyle ou nitro.

La réaction de l'ammoniac sur l'iminoéther s'effectue généralement dans un solvant organique tel qu'un alcool (méthanol, éthanol par exemple) ou dans un éther (dioxanne, glyme, diglyme par exemple), à une température comprise entre 0 et 50°C.

L'action du produit de formule générale (IV) s'effectue le cas échéant dans un solvant tel qu'un amide (diméthylformamide, hexaméthylphosphorotriamide, ou diméthylacétamide), un nitrile (acétonitrile), une cétone (acétone), un dérivé nitré (nitrométhane, nitrobenzène) ou la N-méthylpyrrolidone, à une température comprise entre 0 et 60°C.

L'iminoéther de formule générale (III) peut être obtenu par traitement en milieu acide, d'un nitrile de formule générale :

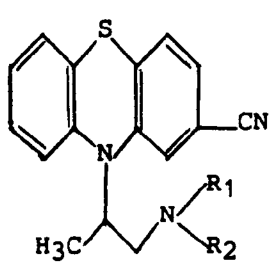

(V)

par un alcool de formule générale :

$$R'OH \qquad (VI)$$

dans laquelle R' est défini comme précédemment.

Généralement, on opère en présence d'acide chlorhydrique, à une température comprise entre -10 et 60°C. Il n'est pas indispensable d'isoler l'iminoéther de formule générale (III) pour le mettre en oeuvre dans la préparation des dérivés de la phénothiazine de formule générale (I).

Le nitrile de formule générale (V) peut être obtenu selon le schéma suivant :

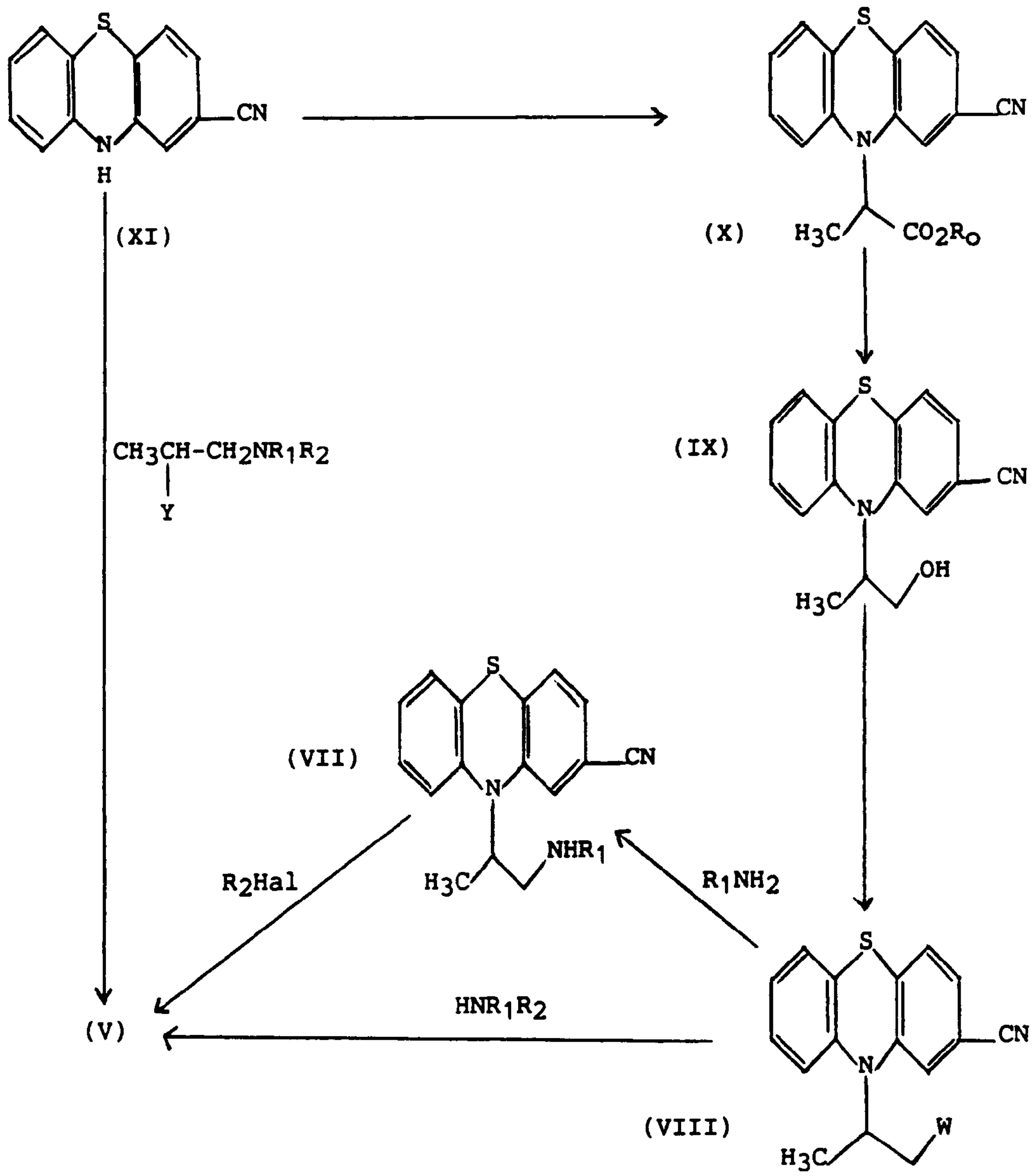


dans lequel Y est un atome d'halogène choisi parmi le chlore, le brome ou l'iode ou un reste p-toluènesulfonyloxy ou méthylsulfonyloxy, W est un reste p.toluènesulfonyloxy, méthylsulfonyloxy ou diaryloxyphosphoryloxy et $R_o$ est un radical alcoyle contenant 1 à 4 atomes de carbone (éthyle par exemple). Les conditions opératoires sont définies plus en détail ci-après dans les exemples.

Le nitrile de formule générale (XI) peut être obtenu comme décrit dans le brevet américain 2 877 224.

Les isomères des produits de formule générale (I) peuvent être obtenus selon les méthodes connues.

On opère notamment par préparation de l'isomère du dérivé de la phénothiazine de formule générale (IX) qui est ensuite transformé en un dérivé de la phénothiazine de formule générale (I) par les méthodes décrites précédemment.

Le dérivé optiquement actif du produit de formule générale (IX) est notamment obtenu par préparation de l'ester d'un diacide, formation d'un sel optiquement actif, séparation des isomères par cristallisation et saponification de l'isomère obtenu.

Plus particulièrement l'ester est obtenu au moyen de l'anhydride d'un diacide comme par exemple l'anhydride phtalique, l'anhydride maléique ou succinique. Le sel est formé par addition d'une amine optiquement active, par exemple la (+) phényl-1 éthylamine, ou la (-) phényl-1 éthylamine.

Dans les exemples qui suivent, on appelle série D les dérivés de la phénothiazine préparés à partir de l'alcool de formule générale (IX) pour lequel le pouvoir rotatoire en solution dans le chloroforme est positif; on

appelle série L les dérivés de la phénothiazine préparés à partir de l'alcool de formule générale (IX) pour lequel le pouvoir rotatoire en solution dans le chloroforme est négatif.

Les produits de formule générale (I) peuvent être purifiés par chromatographie ou cristallisation.

Les dérivés de la phénothiazine de formule générale (I) peuvent être transformés en sels d'addition avec les acides, par action d'un acide dans un solvant organique tel qu'un alcool, une cétone, un ester, un éther ou un solvant chloré. Le sel précipite, éventuellement après concentration de sa solution, il est séparé par filtration ou décantation.

Comme sels pharmaceutiquement acceptables, on peut citer les sels d'addition avec les acides minéraux tels que chlorhydrates, bromhydrates, sulfates, nitrates, phosphates, ou organiques tels que acétates, propionates, succinates, maléates, fumarates, méthanesulfonates, p.toluènesulfonates, iséthionates ou des dérivés de substitution de ces composés.

Les dérivés de la phénothiazine de formule générale (I) présentent une activité antidiarrhéique particulièrement intéressante du fait de leur affinité puissante pour les récepteurs Mu et de leur faible toxicité.

Ils se sont en effet montrés actifs à des concentrations comprises entre 0,05 et 50 nM dans la méthode de binding à la [D-Ala, MePhe, Gly-ol] enképhaline tritiée (DAGO tritié) dans des homogénats de cerveau de cobaye, inspirée de la technique de R. Cotton, H.W. Kosterlitz et coll., Br. J. Pharmac., 84, 927 (1985).

Ils se sont également montrés actifs chez la souris, dans la technique de détermination de l'activité antidiarrhéique inspirée de la méthode de Niemegeers et coll., Arz. Forsch., 22, 516 (1972). Leur $DA_{50}$ est généralement comprise entre 0,3 et 10 mg/kg par voie sous cutanée.

Par ailleurs la toxicité aigüe ($DL_{50}$) des produits de formule générale (I) chez la souris est peu élevée aux doses d'utilisation. Leur $DL_{50}$ est généralement comprise entre 30 mg/kg s.c. et des doses nettement supérieures à 100 mg/kg s.c.

D'un intérêt particulier sont les produits de formule générale (I) dans laquelle le symbole R représente, soit un radical (IIa) pour lequel $R_1$ et $R_2$ identiques ou différentes sont des radicaux alcoyle contenant 1 ou 2 atomes de carbone ou forment ensemble avec l'atome d'azote auquel ils sont rattachés un hétérocycle à 5 ou 6 chaînons éventuellement substitué par 1 ou 2 radicaux méthyle, soit R représente un radical (IIb) pour lequel $R_1$ et $R_2$ sont définis comme ci-dessus et $R_3$ représente un radical alcoyle contenant 1 ou 2 atomes de carbone.

Et parmi ces produits, plus spécialement actifs sont les produits pour lesquels le symbole R est défini comme ci-dessus, sous forme de mélange d'isomères ou sous forme D.

Notamment les produits suivants :

- La [(pyrrolidinyl-1)-1 propyl-2]-10 phénothiazinecarboxamidine-2;
- La [(méthyl-2 pyrrolidinyl-1)-1 propyl-2]-10 phénothiazinecarboxamidine-2 ;
- La [(diméthyl-3,3 pipéridyl-1)-1 propyl-2]-10 phénothiazinecarboxamidine-2 ;
- L'iodure de [méthyl-1 pyrrolidinio-1)-1 propyl-2]-10 phénothiazinecarboxamidine-2.

Les exemples suivants, donnés à titre non limitatif, illustrent la présente invention.

## EXEMPLE 1

On fait barbotter pendant 4 heures de l'ammoniac dans une solution de 13,63 g de dichlorhydrate de [(pyrrolidinyl-1)-1 propyl-2-(2RS)]-10 phénothiazinecarboximidate-2 d'éthyle dans 150 cm3 d'éthanol absolu contenant 8,4 cm3 de triéthylamine. Le mélange réactionnel est agité pendant 24 heures à 25°C. La suspension obtenue est alors filtrée et le filtrat est concentré à sec à 50°C sous pression réduite (5 mm de Hg; 0,67 kPa) pour donner un solide jaune qui est purifié par chromatographie sur colonne (hauteur : 23 cm; diamètre : 4 cm) d'alumine en éluant par des mélanges de chlorure de méthylène et de méthanol 95-5 (en volumes) (750 cm3) et 50-50 (en volumes) (1500 cm3) et en recueillant des fractions de 125 cm3. Les fractions 8 à 11 sont réunies et concentrées à sec à 50°C sous pression réduite (5 mm de Hg; 0,67 kPa) pour donner 4,5 g de chlorhydrate de [(pyrrolidinyl-1)-1 propyl-2-(2RS)]-10 phénothiazinecarboxamidine-2 sous la forme d'un solide jaune.

On porte au reflux une solution de 4,3 g de chlorhydrate de [(pyrrolidinyl-1)-1 propyl-2-(2RS)]-10 phénothiazinecarboxamidine-2 dans 150 cm3 d'acétone. La solution est filtrée sur verre fritté et le filtrat est amorcé par grattage. Le mélange est agité pendant 24 heures à 25°C puis essoré, et le solide est lavé par 10 cm3 d'acétone glacé et séché à 60°C sous pression réduite (1 mm de Hg; 0,13 kPa) pour donner 3,35 g de chlorhydrate de [(pyrrolidinyl-1)-1 propyl-2-(2RS)]-10 phénothiazinecarboxamidine-2 sous la forme de cristaux jaunes fondant à 228-230°C.

RMN du proton (250 MHz, DMSO, $\delta$ en ppm, J en Hz).

1,65 (D, J = 7, 3H, $-CH_3$); 1,7 (Mf, 4H, $-CH_2-CH_2-$ de la pyrrolidine); 2,53 (Mt en partie masqué, $>N-CH_2-$ de la pyrrolidine); 2,86 (DD, J = 13 et 6,5, 1H, 1H du $>NCH_2-$); 3,04 (DD, J = 13 et 6, 1H, 1H du $>N-CH_2-$); 4,28 (Mt, J = 7 - 6,5 et 6, 1H, $>N-CH<$); 6,95 à 7,3 (Mt, 4H, aromatiques); 7,35 (AB limite, 2H, H en 3 et H en 4); 7,45 (S, 1H, -H en 1); 9,28 (Mf, environ 3H,

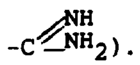

Spectre infra-rouge (KBr), bandes caractéristiques en $cm^{-1}$ :
3240, 3070, 1665, 1595, 1525, 1460, 1420, 870, 830, 735.

Le dichlorhydrate de [(pyrrolidinyl-1)-1 propyl-2-(2RS)]-10 phénothiazinecarboximidate-2 d'éthyle peut être préparé de la manière suivante :

A une solution de 25,16 g de [(pyrrolidinyl-1)-1 propyl-2-(2RS)]-10 phénothiazinecarbonitrile-2 dans 150 cm3 d'éthanol à 40°C, on fait barbotter de l'acide chlorhydrique gazeux pendant 5 heures. La solution est concentrée à sec à 50°C sous pression réduite (30 mm de Hg; 4 kPa) pour donner 38,7 g de dichlorhydrate de [pyrrolidinyl-1)-1 propyl-2-(2RS)]-10 phénothiazinecarboximidate-2 d'éthyle sous la forme d'une meringue marron.

Le [(pyrrolidinyl-1)-1 propyl-2-(2RS)]-10 phénothiazinecarbonitrile-2 peut être obtenu de la manière suivante :

Un mélange de 10 g de méthanesulfonate de (cyano-2 phénothiazinyl-10)-2 propyle-1-(2RS) et de 11,6 cm3 de pyrrolidine dans 50 cm3 de toluène est porté à 90°C sous agitation pendant 24 heures. Après refroidissement, on concentre à sec sous pression réduite (30 mm de Hg; 4 kPa) à 40°C et on reprend le résidu avec 200 cm3 d'éther éthylique et 15 cm3 d'une solution aqueuse de soude 4N. Après avoir agité pendant 10 minutes, on décante et lave la phase organique avec 3 fois 25 cm3 d'une solution aqueuse saturée de chlorure de sodium, sèche sur sulfate de magnésium, filtre et concentre à sec sous pression réduite (30 mm de Hg; 4 kPa) à 20°C. Les 11,3 g d'huile résiduelle sont dissous dans 60 cm3 d'une solution aqueuse d'acide chlorhydrique 0,5N. Cette solution est lavée avec 100 cm3 d'éther éthylique puis alcalinisée avec un excès d'une solution aqueuse de soude N et extraite par 100 cm3 d'éther éthylique. La phase organique est lavée avec 50 cm3 d'une solution aqueuse saturée de chlorure de sodium, séchée sur sulfate de magnésium, filtrée et concentrée à sec sous pression réduite (30 mm de Hg; 4 kPa) à 20°C. L'huile jaune résiduelle (9,5 g) est purifiée sur une colonne (hauteur : 30 cm; diamètre : 5,8 cm) de gel de silice (0,04-0,063 mm) sous légère surpression d'azote (40 kPa) en éluant avec un litre d'un mélange de chlorure de méthylène et de méthanol (95-5 en volumes) et en recueillant des fractions de 100 cm3. Les fractions 3 à 10 sont réunies et concentrées à sec sous pression réduite (30 mm de Hg; 4 kPa) à 40°C. On obtient ainsi 5,45 g de [(pyrrolidinyl-1)-1 propyl-2-(2RS)]-10 phénothiazine carbonitrile-2 sous forme d'une huile jaune.

Le méthanesulfonate de (cyano-2 phénothiazinyl-10)-2 propyle-1-(2RS) peut être obtenu de la manière suivante :

Sur une solution de 120,5 g d'[hydroxy-1 propyl-2-(2RS)]-10 phénothiazinecarbonitrile-2 dans 1280 cm3 de chlorure de méthylène, refroidie à une température voisine de 5°C, on verse, sous agitation, 100 cm3 de triéthylamine puis, en 30 minutes, 55,9 cm3 de chlorure de méthanesulfonyle et on poursuit l'agitation pendant 15 minutes en maintenant la température vers 10-15°C. Le mélange réactionnel est dilué avec 500 cm3 d'eau distillée à 5°C et la phase organique est séparée, lavée avec 500 cm3 d'une solution aqueuse saturée de chlorure de sodium, séchée sur sulfate de sodium, filtrée et concentrée à sec sous pression réduite (30 mm de Hg; 4 kPa) à 40°C. L'huile résiduelle (164 g) est purifiée par chromatographie sur une colonne (hauteur : 54 cm; diamètre : 8,5 cm) de gel de silice (0,2-0,063 mm) en éluant par 4,4 litres de chlorure de méthylène puis par 7 litres d'un mélange de chlorure de méthylène et de méthanol (99-1 en volumes) et en recueillant des fractions de 1 litre. Les fractions 3 à 11 sont réunies et concentrées à sec sous pression réduite (30 mm de Hg; 4 kPa) à 40°C. On obtient ainsi 153,5 g d'une huile jaune que l'on reprend par 400 cm3 d'oxyde d'isopropyle au reflux. Par refroidissement, un produit cristallise. L'agitation est poursuivie pendant 1 heure à une température voisine de 5°C. Le solide formé est essoré, lavé par 2 fois 50 cm3 d'oxyde d'isopropyle glacé et séché à 30°C sous pression réduite (30 mm de Hg; 0,4 kPa). On obtient ainsi 131,6 g de méthanesulfonate de (cyano-2 phénothiazinyl-10)-2 propyle-1-(2RS) sous forme de cristaux jaune clair fondant à 124°C.

L'[hydroxy-1 propyl-2-(2RS)]-10 phénothiazinecarbonitrile-2 peut être préparé de la manière suivante :

Dans une suspension de 52 g de borohydrure de sodium dans 1,4 litre de tétrahydrofuranne, on verse, sous agitation en 15 minutes et à une température voisine de 20°C, 113 cm3 d'éthanedithiol-1,2 puis, en 15 minutes dans les mêmes conditions, une solution de 296 g de (cyano-2 phénothiazinyl-10)-2 propionate d'éthyle-(2RS) dans 1,4 litre de tétrahydrofuranne. A la fin de l'addition, on chauffe le mélange réactionnel pendant 20 heures à une température voisine de 60°C. Après refroidissement à une température de 5°C, on verse 1 litre d'une solution aqueuse de soude 4N pendant 1 heure : un vif dégagement gazeux est observé. Le mélange réactionnel est ensuite versé dans un mélange de 1 litre d'une solution aqueuse de soude 4N et de 3 litres de chlorure de méthylène sous agitation. On isole la phase organique et on extrait à nouveau la phase aqueuse avec 1 litre de chlorure de méthylène. Les phases organiques réunies sont lavées par 2 fois 1 litre d'une solution aqueuse saturée de chlorure de sodium, séchées sur sulfate de magnésium, filtrées et concentrées à sec sous pression réduite (30 mm de Hg; 4 kPa) à 30°C. L'huile visqueuse orangée (290 g) est purifiée sur une colonne (hauteur : 50 cm; diamètre : 8,5 cm) de gel de silice (0,2-0,063 mm) en éluant successivement par 3 litres de chlorure de méthylène, puis par 4 litres d'un mélange de chlorure de méthylène et de méthanol (97,5-2,5 en volumes) et par 10 litres un mélange de chlorure de méthylène et de méthanol (95-5 en volumes) et en recueillant des fractions de 1 litre. Les fractions 3 à 15 sont réunies et concentrées à sec sous pression réduite (30 mm de Hg; 4 kPa) à 30°C. On obtient ainsi 169,7 g d'[hydroxy-1 propyl-2-(2RS)]-10 phénothiazinecarbonitrile-2 sous forme d'une solide jaune fondant à 123°C.

Le (cyano-2 phénothiazinyl-10)-2 propionate d'éthyle-(2RS) peut être préparé de la manière suivante :

Dans une suspension de 24 g d'hydrure de sodium dans 1 litre de diméthylformamide à une température voisine de 25°C, on verse, sous agitation et en 2 heures 30 minutes, une solution de phénothiazinecarbonitrile-2 (224,5 g) dans 1 litre de diméthylformamide puis on laisse encore agiter 1 heure 15 minutes jusqu'à fin de dégagement gazeux. La suspension fine obtenue est versée, sous agitation et à une température voisine de 25°C, en 4 heures 30 minutes dans une solution de 255 cm3 de chloro-2 propionate d'éthyle dans 1 litre de diméthylformamide et on poursuit l'agitation pendant 16 heures. On verse alors, dans le mélange réactionnel, 100 cm3 d'éthanol, puis on verse le tout sur un mélange de 2 kg de glace dans 4 litres d'eau distillée : une gomme précipite puis cristallise. On essore le solide formé, le lave successivement avec 6 fois 500 cm3 d'eau distillée et 2 fois 500 cm3 d'éther de pétrole et le sèche à l'air. On obtient ainsi 296,5 g de (cyano-2 phénothiazinyl-10)-2 propionate d'éthyle-(2RS) sous la forme de cristaux kaki fondant à 117-8°C, utilisé tel quel à l'étape suivante.

EXEMPLE 2

On fait barbotter de l'acide chlorhydrique gazeux dans une solution de 5 g de [(pyrrolidinyl-1)-1 propyl-2]-10 phénothiazinecarbonitrile-2, série D, dans 100 cm3 d'éthanol absolu pendant 3 heures. Le mélange réactionnel rouge est agité pendant 16 heures à 25°C, purgé par barbottage d'azote pendant 2 heures, refroidi à une température voisine de 0°C puis saturé d'ammoniac par barbottage pendant 6 heures. Le mélange est alors agité à 25°C pendant 16 heures, purgé à l'azote en tiédissant à 40°C pendant 1 heure puis concentré à sec à 50°C sous pression réduite (30 mm de Hg; 4 kPa). Le résidu (8,5 g) est purifié par chromatographie sur colonne (hauteur : 30 cm; diamètre : 3 cm) d'alumine en éluant par du chlorure de méthylène (600 cm3) puis par des mélanges de chlorure de méthylène et de méthanol 95-5 (600 cm3), 90-10 (600 cm3), 50-50 (2 litres) (en volumes) et en recueillant des fractions de 60 cm3. Les fractions 15 à 35 sont réunies et concentrées à sec sous pression réduite (30 mm de Hg; 4 kPa) pour donner un solide qui est repris dans 200 cm3 d'oxyde d'isopropyle bouillant. Par refroidissement à 25°C, un solide jaune cristallise. Ce solide est à nouveau dissous dans 100 cm3 d'acétonitrile bouillant. Par refroidissement, le produit cristallise. Il est essoré, lavé par 2 fois 10 cm3 d'acétonitrile puis par 10 cm3 d'éther éthylique et séché à 30°C sous pression réduite (1 mm de Hg; 0,13 kPa) pour donner 4,5 g de chlorhydrate de [(pyrrolidinyl-1)-1 propyl-2]-10 phénothiazinecarboxamidine-2, série D, sous forme d'une poudre jaune vif fondant à 214-216°C.

$[\alpha]^{20}_{D}$ = -42,2° (c = 1,029%; méthanol).

RMN du proton (250 MHz, DMSO, $\delta$ en ppm, J en Hz).

1,65 (D, J = 7, 3H, -$CH_3$); 1,7 (Mf, 4H, -$CH_2$-$CH_2$- de la pyrrolidine); 2,53 (Mt masqué, >N-$CH_2$- de la pyrrolidine); 2,87 (DD, J = 12,5 et 6,5, 1H, 1H du >N-$CH_2$-); 3,02 (DD, J = 12,5 et 6, 1H, 1H du >N$CH_2$-); 4,29 (Mt, J = 7, 6,5 et 6, 1H, >N-CH<); 6,95 à 7,30 (Mt, 4H, aromatiques); 7,35 (AB limite, 2H, -H en 3 et -H en 4); 7,45 (S, 1H, -H en 1); 9,20 (Mf, 3H,

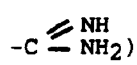
).

Spectre infra-rouge (KBr), bandes caractéristiques en $cm^{-1}$ :

3250, 3060, 1670, 1595, 1525, 1460, 1415, 870, 820, 750.

Le [(pyrrolidinyl-1)-1 propyl-2)-10 phénothiazinecarbonitrile-2, série D, peut être préparé de la manière suivante :

Un mélange de 25,5 g de méthanesulfonate de (cyano-2 phénothiazinyl-10)-2 propyle-1, série D, et de 29,6 cm3 pyrrolidine dans 260 cm3 de toluène est chauffé pendant 52 heures à une température voisine de 90°C. Le mélange réactionnel est concentré à sec sous pression réduite (30 mm de Hg; 4 kPa) à 40°C. Le résidu est repris par 500 cm3 d'éther éthylique et extrait par 2 fois 100 cm3 d'une solution aqueuse 2N d'acide méthanesulfonique. La phase aqueuse est alcalinisée par de la lessive de soude (d = 1,33) jusqu'à pH 13 à une température voisine de 5°C et extraite successivement par 100 cm3 d'eau distillée et par 100 cm3 d'une

solution aqueuse saturée de chlorure de sodium, séchées sur sulfate de magnésium en présence de noir 3S, filtrées et le filtrat jaune est concentré à sec sous pression réduite (30 mm de Hg; 4 kPa) à 40°C. Les 18,2 g d'huile orangée ainsi obtenus sont chromatographiés sur une colonne (hauteur : 45 cm; diamètre : 4 cm) de gel de silice (0,063-0,2 mm) en éluant par 2,5 litres d'un mélange de chlorure de méthylène et de méthanol (97,5-2,5 en volumes) et en recueillant des fractions de 150 cm3. Les fractions 8 à 16 sont réunies et concentrés à sec sous pression réduite (30 mm de Hg; 4 kPa) à 40°C. On obtient ainsi 11,7 g de [(pyrrolidinyl-1)-1 propyl-2]-10 phénothiazinecarbonitrile-2, série D, sous forme d'une huile jaune.

$[\alpha]_D^{20}$ = -9,8 ±0,4° (1,1%; chloroforme).

Le méthanesulfonate de (cyano-2 phénothiazinyl-10)-2 propyle-1, série D, peut être préparé de la manière suivante :

A une solution de 20 g d'(hydroxy-1 propyl-2)-10 phénothiazinecarbonitrile, série D, dans 200 cm3 de chlorure de méthylène refroidie à une température voisine de 5°C, on ajoute, sous agitation, 16,2 cm3 de triéthylamine puis on verse, goutte à goutte pendant 25 minutes, une solution de 8,9 cm3 de chlorure de méthane sulfonyle dans 89 cm3 de chlorure de méthylène et poursuit l'agitation pendant 50 minutes à une température voisine de 10°C. Le mélange réactionnel est lavé successivement par 2 fois 100 cm3 d'eau distillée et par 100 cm3 d'une solution aqueuse saturée de chlorure de sodium, séché sur sulfate de magnésium, filtré et concentré à sec sous pression réduite (30 mm de Hg; 4 kPa) à 40°C. On obtient ainsi 25,8 g de méthanesulfonate de (cyano-2 phénothiazinyl-10)-2 propyle-1, série D, sous forme d'une gomme orangée qui est utilisée sans autre purification pour la suite des synthèses.

$[\alpha]_D^{20}$ = -23,1 ±0,4° (1,4%; chloroforme).

L'(hydroxy-1 propyl-2)-10 phénothiazinecarbonitrile-2, série D, peut être préparé de la manière suivante :

A une solution de 23,5 g de potasse dans 1150 cm3 d'éthanol au reflux, on ajoute 95,4 g de phtalate de (-)-(cyano-2 phénothiazinyl-10)-2 propyle-1, et de phényl-1 éthylammonium-(S) et poursuit le reflux sous agitation pendant 10 minutes. Le mélange réactionnel est alors versé sur 1 litre d'eau glacée et extrait par 2 litres puis 500 cm3 d'acétate d'éthyle. Les phases organiques réunies sont lavées successivement par deux fois 500 cm3 d'une solution aqueuse d'acide chlorhydrique 0,1N, par 2 fois 500 cm3 d'une solution aqueuse saturée d'hydrogénocarbonate de sodium et par 500 cm3 d'une solution aqueuse saturée de chlorure de sodium, séchées sur sulfate de magnésium, filtrées et concentrées à sec sous pression réduite (30 mm de Hg; 4 kPa) à 40°C. Le solide jaune résiduel (44 g) est repris par 200 cm3 d'oxyde d'isopropyle au reflux et le produit cristallise à chaud. On laisse revenir à une température voisine de 20°C et on essore le solide, le lave par 20 cm3 d'oxyde d'isopropyle et le sèche sous pression réduite (5 mm de Hg; 0,7 kPa) à 20°C. On obtient ainsi 36,5 g d'(hydroxy-1 propyl-2)-10 phénothiazinecarbonitrile-2, série D, sous forme de cristaux jaunes fondant à 135°C.

$[\alpha]_D^{20}$ = +13,1 ±0,5° (1,0%; chloroforme).

Le phtalate de (-)-(cyano-2 phénothiazinyl-10)-2 propyle et de phényl-1 éthylammonium-(1S) peut être préparé de la manière suivante :

On porte à reflux pendant 6 heures, sous agitation, une suspension de 56,5 g d'[(hydroxy-1 propyl-2-(2RS)]-10 phénothiazinecarbonitrile-2 et de 32,6 g d'anhydride phtalique dans 100 cm3 de pyridine anhydre. Après refroidissement, le mélange réactionnel est dilué par 500 cm3 de chlorure de méthylène, lavé par 4 fois 100 cm3 d'eau distillée, séché sur sulfate de magnésium, filtré et concentré à sec sous pression réduite (30 mm de Hg; 4 kPa) à 40°C. Le résidu est agité avec 500 cm3 d'une solution aqueuse N d'acide chlorhy- drique puis décanté et dissous dans 500 cm3 d'acétate d'éthyle. La solution est lavée par 2 fois 100 cm3 d'une solution aqueuse N d'acide chlorhydrique puis par 100 cm3 d'une solution aqueuse de chlorure de sodium. La phase organique est séchée sur sulfate de magnésium, filtrée et concentrée à sec sous pression réduite (30 mm de Hg; 4 kPa) à 50°C. On obtient ainsi 102 g d'une huile épaisse contenant de l'acide [cyano-2 phénothiazinyl-10)-2 propyl-1-(2RS)] oxycarbonyl-2 benzènecarboxylique et utilisés tels quels ultérieurement.

On dissout 102 g de l'huile précédemment obtenue et contenant l'acide [(cyano-2 phénothiazinyl-10)-2 propyl-1-(2RS)]oxycarbonyl-2 benzènecarboxylique dans 500 cm3 d'acétate d'éthyle et on ajoute, sous agitation, à une température voisine de 20°C, une solution de 24,2 g de (-)-phényl-1 éthylamine-(1S) dans 360 cm3 d'acétate d'éthyle. Après 2 jours d'agitation à une température voisine de 20°C, le solide formé est filtré et dissous dans 600 cm3 d'acétate d'éthyle au reflux. Après refroidissement, le solide formé est essoré, lavé par 50 cm3 d'acétate d'éthyle et séché sous pression réduite (30 mm de Hg; 4 kPa) à 40°C. On obtient ainsi 44,2 g de phtalate de (-)-(cyano-2 phénothiazinyl-10)-2 propyle et de phényl-1 éthylammonium-(1S) sous forme de cristaux jaunes clairs fondant à 154°C.

$[\alpha]_D^{20}$ = -21,5° ±0,6° (1%; chloroforme).

EXEMPLE 3

Une solution de 0,52 g de [(méthyl-2 pyrrolidinyl-1-(2RS))-1 propyl-2]-10 phénothiazinecarbonitrile-2, série D, dans 10 cm3, d'éthanol est saturée d'acide chlorhydrique gazeux par barbottage pendant 3 heures. Le mélange est agité pendant 12 heures à 25°C, purgé par barbottage d'azote pendant 2 heures puis saturé par barbottage d'ammoniac pendant 10 heures. La suspension est purgée par barbottage d'azote, filtrée et le filtrat est concentré à sec sous pression réduite à 50°C (30 mm de Hg; 4 kPa) pour donner un résidu qui est purifié par chromatographie sur colonne (hauteur: 30 cm; diamètre : 1,5 cm) d'alumine en éluant par du chlorure de méthylène pur (200 cm3) puis par des mélanges de chlorure de méthylène et de méthanol 90-10 (100 cm3) et 50-50 (200cm3) (en volumes) en recueillant des fractions de 20 cm3. Les fractions 15 à 20 sont réunies et concentrées à sec sous pression réduite à 50°C (30 mm de Hg; 4 kPa) pour donner 0,7 g d'un solide jaune d'or qui est repris dans 10 cm3 d'oxyde d'isopropyle bouillant. Après grattage, une cristallisation se développe. La suspension est ramenée à 25°C, sous agitation, pour donner 0,53 g de chlorhydrate de [(méthyl-2 pyrrolidinyl-1-(2RS))-1 propyl-2]-10 phénothiazinecarboxamidine-2, série D sous la forme de cristaux jaunes fondant à environ 75°C.

RMN du proton (250 MHz, DMSO, $\delta$ en ppm, J en Hz).

0,63 (D, J = 6, 3H, $-CH_3$ du méthyl-2 pyrrolidinyle); 1,24 et 1,5 à 1,90 (2Mt, respectivement 1H et 3H, $-CH_2CH_2-$ du méthyl-2 pyrrolidinyle); 1,67 (D, J = 7, 3H, $-CH_3$); 2,08 (Mt, 1H, 1H du >$NCH_2$- du méthyl-2 pyrrolidinyle); 2,25 (Mt, 1H, >N-CH< du méthyl-2 pyrrolidinyle); 2,27 (DD, J = 13,5 et 5, 1H, 1H du >N-$CH_2$-); 3,08 (Mt, 1H, 1H du >N-$CH_2$- du méthyl-2 pyrrolidinyle); 3,35 (DD, J = 13,5 et 6,5, 1H, 1H du >N-$CH_2$-); 4,24 (Mt, J = 7 - 6,5 et 5, 1H, >N-CH<); 6,90 à 7,25 (Mt, 4H, aromatiques); 7,32 (AB limite, 2H, -H en 3 et -H en 4); 7,43 (S, 1H, -H en 1); 9,20 (Mf, 4H

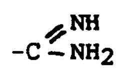

HCl).

Spectre infra-rouge (KBr), bandes caractéristiques en $cm^{-1}$ :3240, 3060, 1670, 1595, 1525, 1460, 1415, 870, 820, 750.

Une solution de 6,8 g de méthanesulfonate de (cyano-2 phénothiazinyl-10)-2 propyle, série D, dans 80 cm3 de toluène et 3,5 cm3 de méthyl-2-(2RS) pyrrolidine est chauffée sous agitation à 100°C pendant 48 heures. Après refroidissement à 25°C, le mélange est transféré dans une ampoule à décanter où il est lavé par 2 fois 200 cm3 d'eau distillée et 50 cm3 de saumure. La phase organique est séchée sur sulfate de magnésium, filtrée et le filtrat est concentré à sec sous pression réduite (30 mm de Hg; 4 kPa) pour donner un résidu qui est purifié par chromatographie sur colonne (hauteur : 35 cm; diamètre : 3,2 cm) de silice (0,2-0,06 mm) en éluant par un mélange de cyclohexane et d'acétate d'éthyle 70-30 (1 litre) (en volumes) et en recueillant des fractions de 60 cm3. Les fractions 11 à 14 sont réunies et concentrées à sec sous pression réduite (30 mm de Hg; 4 kPa) pour donner 0,5 g de [(méthyl-2 pyrrolidinyl-1-(2RS))-1 propyl-2]-10 phénothiazinecarbonitrile-2, série D, sous la forme d'une huile jaune.

RMN du proton (250 MHz, DMSO, $\delta$ en ppm, J en Hz).

0,73 (D, J = 6, 3H, $-CH_3$ du méthyl-2 pyrrolidinyle); 1,24 et 1,5 à 1,9 (2Mt, respectivement 1H et 3H, $-CH_2$- du méthyl-2 pyrrolidinyle); 1,62 (D, J = 7,5, 3H, $-CH_3$); 2,09 (Mt, 1H, 1H du >$NCH_2$- du méthyl-2 pyrrolidinyle); 2,27 (Mt, 1H, >N-CH< du méthyl-2 pyrrolidinyle); 2,37 (DD, J = 13,5 et 5, 1H, 1H du >N-$CH_2$-); 3,11 (Mt, 1H, 1H du >N-$CH_2$- du méthyl-2 pyrrolidinyle); 3,32 (DD, J = 13,5 et 7, 1H, 1H du>N-$CH_2$-); 4,10 (Mt, J = 7,5 - 7 et 5, 1H, N-CH ); 6,95 à 7,25 (Mt, 4H, aromatiques); 7,27 (D, J = 7,5, 1H, -H en 4); 7,36 (DD, J = 7 et 2, 1H, -H en 3); 7,57 (D, J = 2, 1H, -H en 1).

EXEMPLE 4

Dans une solution de 8,3 g de [(diméthyl-3,3 pipéridyl-1)-1 propyl-2]-10 phénothiazinecarbonitrile-2, série D, dans 83 cm3 d'éthanol absolu, on fait barbotter de l'acide chlorhydrique pendant 5 heures à une température voisine de 45°C. La solution obtenue est agitée pendant 16 heures à 25°C, purgée par barbottage d'azote, refroidie à 0°C et saturée d'ammoniac en maintenant la température vers 5°C pendant 6 heures. Le mélange réactionnel est agité pendant 16 heures à 25°C, filtré et concentré à sec sous pression réduite (30 mm de Hg; 4 kPa) à 50°C pour donner 12,5 g d'un solide jaune. Ce solide est repris par 100 cm3 de chlorure de méthylène. Le mélange est filtré et le filtrat est concentré à sec sous pression réduite (30 mm de Hg; 4 kPa) à 50°C pour donner 10 g de chlorhydrate de [(diméthyl-3,3 pipéridyl-1)-1 propyl-2]-10 phénothiazinecarboxamidine-2, série

D, sous forme d'un solide amorphe jaune.

On dissout 7,8 g de chlorhydrate de [(diméthyl-3,3 pipéridyl-1)-1 propyl-2]-10 phénothiazinecarboxamidine-2, série D, dans 400 cm3 d'acétonitrile bouillant. Après un léger refroidissement à 70°C, la cristallisation se développe. La suspension est agitée pendant 12 heures à 25°C. Le solide est essoré sur verre fritté et séché à 60°C sous pression réduite (1 mm de Hg; 0,13 kPa) pour donner 4,49 g de chlorhydrate de [(diméthyl-3,3 pipéridyl-1)-1 propyl-2]-10 phénothiazinecarboxamidine-2, série D, sous la forme de cristaux jaune d'or fondant à 256-260°C.

$[\alpha]^{20}_{D}$ = -32° (c = 1%; acide méthanesulfonique 0,1N).

RMN du proton (250 MHz, DMSO δ en ppm, J en Hz).

0,81 et 0,84 (2S, 6H, $-CH_3$ du diméthyl-3,3 pipéridyle); 1,15 et 1,52 (2Mt, 2H pour chaque, $-CH_2$- du diméthyl pipéridyle); 1,65 (D, J = 7, 3H, $-CH_3$); 2 (S, 2H, >$NCH_2$- en 2 du diméthyl-3,3 pipéridyle); 2,15 et 2,42 (2Mf, 1H chacun, >$NCH_2$- en 6 du diméthyl pipéridyle); 2,50 (DD en partie masqué, 1H du >$NCH_2$-); 2,92 (DD, J = 13,5 et 7,5, 1H, 1H du >N-$CH_2$-); 4,32 (Mt, J = 7,5 - 7 et 6,5, 1H, >N-CH<); 6,95 à 7,25 (Mt, 4H, aromatiques); 7,34 (AB limite, 2H, -H en 3 et -H en 4) ; 7,4 (S, 1H, -H en 1) ; 9,4 (Mf, 4H,

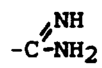 ) .

Spectre infra-rouge (KBr), bandes caractéristiques en $cm^{-1}$ : 3240, 3060, 2975, 2940, 1665, 1595, 1525, 1460, 1415, 870, 810, 730.

Le [diméthyl-3,3 pipéridyl-1)-1 propyl-2]-10 phénothiazinecarbonitrile-2, série D, peut être obtenu de la manière suivante :

A une solution de 10,82 g de méthanesulfonate de (cyano-2 phénothiazinyl-10)-2 propyle, série D, dans 40 cm3 de toluène, on ajoute 17 g de diméthyl-3,3 pipéridine et on chauffe la solution obtenue à 90°C pendant 24 heures. Le mélange réactionnel est dilué avec 50 cm3 de toluène, lavé avec 3 fois 50 cm3 d'eau distillée, séché sur sulfate de magnésium, filtré et concentré à sec sous pression réduite (30 mm de Hg; 4 kPa) à 50°C pour donner 11,4 g d'un miel jaune qui est purifié par chromatographie sur colonne (hauteur : 48 cm; diamètre : 3,8 cm) de silice (0,2-0,06 mm) en éluant par un mélange de cyclohexane et d'acétate d'éthyle 80-20 (en volumes) (1 litre) et en recueillant des fractions de 125 cm3. Les fractions 3 à 7 sont réunies et concentrées à sec sous pression réduite (30 mm de Hg; 4 kPa) à 50°C pour donner 8,73 g de [(diméthyl-3,3 pipéridyl-1)-1 propyl-2]-10 phénothiazinecarbonitrile-2, série D, sous forme d'un miel jaune.

$[\alpha]^{20}_{D}$ = +23,7° (c = 1%; méthanol).

RMN du proton (250 MHz, $CDCl_3$, δ en ppm, J en Hz).

0,97 (S, 6H, $-CH_3$ du diméthyl pipéridyle); 1,25 et 1,65 (2Mt, 2H pour chaque, $-CH_2$- du diméthyl pipéridyle); 1,64 (D, J = 7, $-CH_3$); 2,08 (AB limite, 2H, >$NCH_2$- en 2 du diméthyl pipéridyle); 2,27 et 2,47 (2Mf, 1H chacun, >$NCH_2$- en 6 du pipéridyle); 2,59 (DD, J = 13 et 6,0, 1H, 1H du >N-$CH_2$-); 2,97 (DD, J = 13 et 5,5, 1H, 1H du >N-$CH_2$-); 4,16 (Mt, J = 7 - 6 et 5,5, 1H, >N-CH<); 6,9 à 7,3 (Mt, 6H, aromatiques); 7,58 (S, 1H, -H en 1).

Spectre infrarouge ($CHCl_3$), bandes caractéristiques en $cm^{-1}$ : 2930, 2850, 2785, 2230, 1590, 1570, 1550, 1460, 1415, 870, 815.

EXEMPLE 5

En opérant d'une manière analogue à celle décrite dans l'exemple 1 mais en partant de 13,95 g de dichlorhydrate de [(diméthylamino-1 propyl-2-(2RS)]-10 phénothiazinecarboximidate-2 d'éthyle, on obtient 3,52 g de chlohydrate de [(diméthylamino-1 propyl-2-(2RS)]-10 phénothiazinecarboxamidine-2 sous forme de cristaux jaunes fondant à 186-188°C.

RMN du proton (400 MHz, DMSO D6, δ en ppm, J en Hz).

1,68 (D, J = 7, 3H, $-CH_3$) ; 2,34 (S, 6H, $-N(CH_3)_2$) ; 2,81 (DD, J = 12,5 et 6, 1H, 1 H du >N-$CH_2$-) ; 3,04 (DD, J = 12,5 et 6,5, 1H, 1H du >N-$CH_2$-) ; 4,39 (Mt, J = 7, 6,5 et 6, 1H, >N-CH<); 7 à 7,5 (Mt, 7H, aromatiques) ; 9,5 (Mf, 4 H,

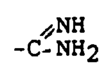,

HCl).

Spectre infra-rouge (KBr), bandes caractéristiques en $cm^{-1}$:
3340, 2000, 1670, 1630, 1590, 1460, 1525, 870, 820, 750, 740.

Le dichlorhydrate de [(diméthylamino-1 propyl-2-(2RS)]-10 phénothiazinecarboximidate-2 d'éthyle peut être préparé de la manière suivante :

En opérant d'une manière analogue à celle décrite dans l'exemple 1 mais en partant de 9,28 g de [(diméthylamino-1 propyl-2-(2RS)]-10 phénothiazinecarbonitrile-2, on obtient 13,95 g de dichlorhydrate de [(diméthylamino-1 propyl-2-(2RS)]-10 phénothiazinecarboximidate-2 d'éthyle sous forme d'une meringue marron.

EXEMPLE 6

En opérant d'une manière analogue à celle décrite dans l'exemple 1 mais en partant de 11,92 g de dichlorhydrate de [(diéthylamino-1 propyl-2-(2RS)]-10 phénothiazinecarboximidate-2 d'éthyle, on obtient 7 g de chlorhydrate de [diéthylamino-1 propyl-2-(2RS)]-10 phénothiazinecarboxamidine-2 sous forme d'une poudre jaune fondant à 175-180°C.

RMN du proton (400 MHz, DMSO D6, δ en ppm, J en Hz).

0,98 (T, J = 7, 6H, -$CH_2\underline{CH_3}$) ; 1,77 (D, J = 7, 3H, -$CH_3$); 2,76 (Mt, 4H, -$\underline{CH_2}CH_3$) ; 2,97 et 3,34 (2 Mf, 1H chacun, >N-$CH_2$-) ; 4,6 (Mf, 1H, >N-CH<) ; 7 à 7,5 (Mt, 7H, aromatiques) ; 9,35 et 9,56 (2 Mf, respectivement 2 H chacun,

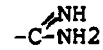,

HCl).

Spectre infra-rouge (KBr), bandes caractéristiques en $cm^{-1}$ : 3240, 2840, 2790, 2660, 2480, 1675, 1630, 1590, 1460, 1525, 870, 820, 750, 735.

Le dichlorhydrate de [(diéthylamino-1 propyl-2-(2RS)]-10 phénothiazinecarboximidate-2 d'éthyle, peut être préparé de la manière suivante :

En opérant d'une manière analogue à celle décrite dans l'exemple 1 mais en partant de 8,44 g de [(diéthylamino-1 propyl-2-(2RS)]-10 phénothiazinecarbonitrile-2, on obtient 11,92 g de dichlorhydrate de [(diéthylamino-1 propyl-2-(2RS)]-10 phénothiazinecarboximidate-2 d'éthyle sous forme d'une meringue orangée.

EXEMPLE 7

A une solution de 0,1 g de chlorhydrate de [(pyrrolidinyl-1)-1 propyl-2-(2RS)]-10 phénothiazinecarboxamidine-2 dans 1 cm3 de dimé- thylformamide, on ajoute 0,036 g d'iodure de méthyle et on agite pendant 16 heures à une température voisine de 20°C. La solution réactionnelle obtenue est versée sur 15 cm3 d'oxyde d'isopropyle et l'huile formée est décantée, lavée avec 15 cm3 d'oxyde d'isopropyle et à nouveau dissoute dans 12 cm3 d'un mélange d'acétone et de méthanol (85-15 en volumes). La solution précédente est versée goutte à goutte, sous agitation dans 50 cm3 d'oxyde d'isopropyle et le solide jaune formé est filtré et séché sous pression réduite (5 mm de Hg; 0,67 kPa) à 50°C. On obtient ainsi 0,11 g de chlorhydrate d'iodure de [(méthyl-1 pyrrolidinio-1)-1 propyl-2-(2RS)]-10 phénothiazinecarboxamidine-2 sous forme d'une poudre jaune fondant au-dessus de 250°C.

RMN du proton (250 MHz, DMSO D6, δ en ppm, J en Hz).

1,93 (D, J = 7, 3H, -$CH_3$) ; 2,03 (Mt, 4H, >$CH_2$ de la pyrrolidine) ; 3,01 (S, 3H, $\rightarrow\overset{N}{+}CH_3I^-$) ; 3,33 et 3,57 (2 Mt, respectivement 1H et 3H, $\rightarrow\overset{N}{+}CH_2$-, $I^-$ de la pyrrolidine) ; 3,79 (DD, J = 12,5 et 1,5, 1H, 1 H du →J $CH_2$-, $I^-$) ; 4,12 (DD, J = 12,5 et 9, 1H, 1 H du $\rightarrow\overset{N}{+}CH_2$-,$I^-$) ; 4,86 (Mt, J = 7,5, 7 et 1,5, 1H, >N-CH<) ; 7,05 à 7,55 (Mt, 7H, aromatiques) ; 8,8 à 9,5 (Mf étalé 4H,

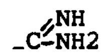,

HCl).

Spectre infra-rouge (KBr), bandes caractéristiques en $cm^{-1}$: 3400, 2500, 1660, 1590, 1560, 1455, 1530, 1515, 860, 825, 750.

EXEMPLE 8

D'une manière analogue à celle décrite à l'exemple 7 mais à partir de 2 g de chlorhydrate de [(pyrrolidinyl-1)-1 propyl-2]-10 phénothiazinecarboxamidine-2, série D, dans 20 cm3 de diméthylformamide et de 0,75 g d'iodure de méthyle, on obtient 1,9 g de chlorhydrate d'iodure de [(méthyl-1 pyrrolidinio-1)-1 propyl-2] -10 phénothiazinecarboxamidine-2, série D, sous la forme d'un poudre jaune.

$[\alpha]_D^{20}$ = -33,4 ± 0,6° (c = 0,856 % ; Méthanol)

RMN du proton (250 MHz, DMSO D6, δ en ppm, J en Hz).

1,94 (D, J = 7, 3H, $-CH_3$) ; 2,04 (Mt, 4H, $-CH_2-$ de la pyrrolidine) ; 3,01 (S, 3H, → $CH_3I^-$) ; 3,35 et 3,58 (2 Mt, respectivement 1 H et 3 H, → $CH_2-$, $I^-$ de la pyrrolidine) ; 3,81 (DD, J = 14, 1 H, 1 H du →$\overset{+}{N}$ $CH_2-,I^-$) ; 4,13 (DD, J = 14 et 9, 1H, 1H du →$\overset{+}{N}$ $CH_2-,I^-$) ; 7, 05 à 7,55 (Mt, 7H, aromatiques) ; 8,96 à 9,34 (2 Mf,

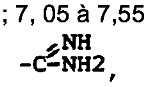

HCl).

Spectre infra-rouge (KBr), bandes caractéristiques en $cm^{-1}$ : 3250, 3085, 1675, 1590, 1560, 1455, 1515, 865, 820, 750

EXEMPLE 9

D'une manière analogue à celle décrite à l'exemple 7 mais à partir de 2,54 g de chlorhydrate de [(diméthylamino-1)-1 propyl-2-(2RS)]-10 phénothiazinecarboxamidine-2, dans 15 cm3 de diméthylformamide et de 0,99 g d'iodure de méthyle, on obtient 0,58 g d'iodure de [triméthylammonio-1 propyl-2-(2RS)]-10 phénothiazinecarboxamidine-2, sous la forme d'une poudre jaune-verdâtre fondant à 248-50°C.

RMN du proton (250 MHz, DMSO D6, δ en ppm, J en Hz).

1,90 (D, J = 7, 3H, $-CH_3$) ; 3,12 (S, 9H, $-\overset{+}{N}(CH_3)_3I^-$) ; 3,77 (D large, J = 14, 1 H, 1 H du →$\overset{+}{N}$ $CH_2-,I^-$) ; 4,07 (DD, J = 14 et 7,5, 1 H, 1 H du →$\overset{+}{N}$ $CH_2-,I^-$) ; 4,83 (Mt, J = 7,5, 7 et 1, 1 H, >N-CH<) ; 7,05 à 7,53 (Mt, 7H, aromatiques).

Spectre infra-rouge (KBr), bandes caractéristiques en $cm^{-1}$ : 3350, 2000, 3000, 1665, 1630, 1590, 1550, 1460, 875, 825, 755.

EXEMPLE 10

D'une manière analogue à celle décrite à l'exemple 7 mais à partir de 2,74 g de chlorhydrate de [(diéthylamino-1)-1 propyl-2-(2RS)]-10 phénothiazinecarboxamidine-2, dans 15 cm3 de diméthylformamide et de 0,99 g d'iodure de méthyle, on obtient 0,38 g d'iodure de [diéthylméthylammonio-1 propyl-2-(2RS)]-10 phénothiazinecarboxamidine-2, sous la forme d'une poudre vert pâle fondant à 254-56°C.

RMN du proton (250 MHz, DMSO D6, δ en ppm J en Hz).

1 à 1,20 (Mt, 6H, $-CH_2C\underline{H_3}$) ; 1,95 (D, J = 7, 3H, $-CH_3$) ; 2,96 (S, 3 H, →$\overset{+}{N}$ $CH_3I^-$) ; 3,42 (Mt masqué, 4H, $>\overset{+}{N}(C\underline{H_2}CH_3)_2I^-$) ; 3,66 (D large, J = 15 et ≤ 1,5, 1 H, 1 H du →$\overset{+}{N}-CH_2-,I^-$) ; 3,97 (DD, J = 15 et 7,5, 1 H, 1 H du →$\overset{+}{N}CH_2-,I^-$) ; 4,82 (Mt, J = 7,5, 7 et ≦ 1,5, 1H, >N-CH<) ; 7,05 à 7,5 (Mt, 7H, aromatiques) ; 7,5 à 9 (Mf étalé, 4H

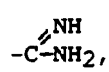

HCl).

Spectre infra-rouge (KBr), bandes caractéristiques en $cm^{-1}$ : 3350, 2000, 1665, 1630, 1590, 1550, 1460, 1520, 870, 820, 755.

La présente invention concerne également les compositions pharmaceutiques qui contiennent les produits de formule générale (I) à l'état pur ou associés à un adjuvant, un diluant et/ou un enrobage compatibles et pharmaceutiquement acceptables. Ces compositions pharmaceutiques peuvent être employées par voie orale.

Comme compositions solides pour administration orale peuvent être utilisés des comprimés, des pilules,

des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions selon l'invention sont particulièrement utiles en thérapeutique humaine pour leur action antidiarrhéique.

Les doses dépendent de l'effet recherché et de la durée du traitement; elles sont généralement comprises entre 0,5 et 25 mg par jour par voie orale pour un adulte.

D'une façon générale, le médecin déterminera la posologie qu'il estime la plus appropriée en fonction de l'âge, du poids et de tous les autres facteurs propres au sujet à traiter.

L'exemple suivant, donné à titre non limitatif, illustre une composition selon l'invention.

EXEMPLE

On prépare, selon la technique habituelle, des comprimés dosés à 1 mg de produit actif ayant la composition suivante :

- chlorhydrate de [(méthyl-2 pyrrolidinyl-1-(2RS)-1 propyl-2]-10 phénothiazinecarboxamidine-2, série D 1,1 mg
- amidon 20 mg
- silice précipité 3,6 mg
- stéarate de magnésium 0,4 mg

## Revendications

### Revendications pour les Etats contractants suivants : AT, BE, CH, DE, FR, GB, IT, LI, LU, NL, SE

**1.** Un dérivé de la phénothiazine de formule générale :

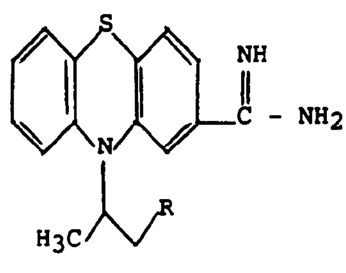

dans laquelle R représente

- soit un radical de formule générale :

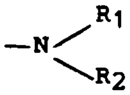

dans laquelle les symboles $R_1$ et $R_2$ identiques ou différents représentent des radicaux alcoyle, hydroxyalcoyle, acétyloxyalcoyle ou forment ensemble avec l'atome d'azote auquel ils sont rattachés, un hétérocycle contenant 4 à 7 chaînons, éventuellement substitué par 1 ou 2 radicaux alcoyle, hydroxyalcoyle ou acétyloxyalcoyle,

- soit un radical de formule générale :

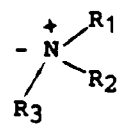

dans laquelle $R_1$ et $R_2$ sont définis comme ci-dessus et $R_3$ représente un radical alcoyle ou phénylalcoyle, étant entendu que les radicaux et portions alcoyle contiennent 1 à 4 atomes de carbone en chaîne droite ou ramifiée, sous ses formes isomères ou leurs mélanges, ainsi que ses sels d'addition avec les acides.

2. Un dérivé de la phénothiazine selon la revendication 1, caractérisé en ce que le symbole R représente soit un radical $-NR_1R_2$ pour lequel $R_1$ et $R_2$ identiques ou différents représentent des radicaux alcoyle contenant 1 ou 2 atomes de carbone ou forment ensemble avec l'atome d'azote auquel ils sont rattachés, un hétérocycle à 5 ou 6 chaînons éventuellement substitué par 1 ou 2 radicaux méthyle, soit R représente un radical $-\overset{+}{N}R_1R_2R_3$ pour lequel $R_1$ et $R_2$ sont définis comme ci-dessus et $R_3$ représente un radical alcoyle contenant 1 ou 2 atomes de carbone, sous ses formes isomères ou leurs mélanges, ainsi que ses sels d'addition avec les acides.

3. Un dérivé de la phénothiazine selon la revendication 1, pour lequel R est défini comme dans la revendication 2, sous forme d'un mélange d'isomères, ou sous forme D, ainsi que ses sels d'addition avec les acides.

4. La [(pyrrolidinyl-1)-1 propyl-2]-10 phénothiazinecarboxamidine-2, sous ses formes isomères et leurs mélanges ainsi que ses sels d'addition avec les acides.

5. La [(méthyl-2 pyrrolidinyl-1)]-1 propyl-2]-10 phénothiazinecarboxamidine-2, sous ses formes isomères et leurs mélanges, ainsi que ses sels d'addition avec les acides.

6. La [(diméthyl-3,3 pipéridyl-1)-1 propyl-2]-10 phénothiazinecarboxamidine-2, sous ses formes isomères et leurs mélanges, ainsi que ses sels d'addition avec les acides.

7. L'iodure de [(méthyl-1 pyrrolidinio-1)-1 propyl-2]-10 phénothiazinecarboxamidine-2, sous ses formes isomères et leurs mélanges, ainsi que ses sels d'addition avec les acides.

8. Procédé de préparation d'un dérivé de la phénothiazine selon la revendication 1, caractérisé en ce que l'on fait agir l'ammoniac sur un iminoéther de formule générale :

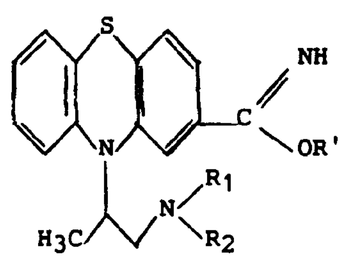

dans laquelle $R_1$ et $R_2$ sont définis comme dans la revendication 1, et R' est un radical alcoyle contenant 1 à 10 atomes de carbone, puis le cas échéant, pour obtenir un produit selon la revendication 1, pour lequel R représente un radical $-\overset{+}{N}R_1R_2R_3$, on fait réagir un produit de formule générale :

$$R_3 - X$$

dans laquelle $R_3$ est défini comme dans la revendication 1 et X représente un atome d'halogène ou un radical alcoylsulfonyloxy ou phénylsulfonyloxy dont le radical phényle est éventuellement substitué par un ou plusieurs atomes d'halogène ou radicaux alcoyle ou nitro, puis transforme éventuellement le produit obtenu en un sel d'addition avec un acide.

9. Composition pharmaceutique caractérisée en ce qu'elle contient au moins un dérivé de la phénothiazine selon la revendication 1, à l'état pur ou sous forme d'une association avec tout autre diluant ou adjuvant

compatible et pharmaceutiquement acceptable.

**Revendication pour les Etats contractants suivants: ES, GR**

1. Procédé de préparation d'un dérivé de la phénothiazine de formule générale :

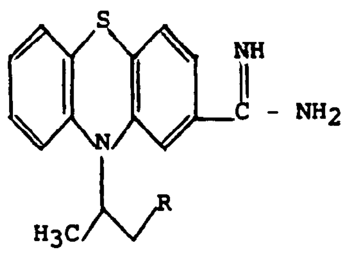

(I)

dans laquelle R représente

- soit un radical de formule générale :

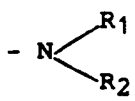

dans laquelle les symboles $R_1$ et $R_2$ identiques ou différents représentent des radicaux alcoyle, hydroxyalcoyle, acétyloxyalcoyle ou forment ensemble avec l'atome d'azote auquel ils sont rattachés, un hétérocycle contenant 4 à 7 chaînons, éventuellement substitué par 1 ou 2 radicaux alcoyle, hydroxyalcoyle ou acétyloxyalcoyle,

- soit un radical de formule générale :

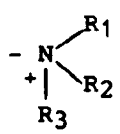

dans laquelle $R_1$ et $R_2$ sont définis comme ci-dessus et $R_3$ représente un radical alcoyle ou phénylalcoyle, étant entendu que les radicaux et portions alcoyle contiennent 1 à 4 atomes de carbone en chaîne droite ou ramifiée, sous ses formes isomères ou leurs mélanges, ainsi que ses sels d'addition avec les acides, caractérisé en ce que l'on fait agir l'ammoniac sur un iminoéther de formule générale :

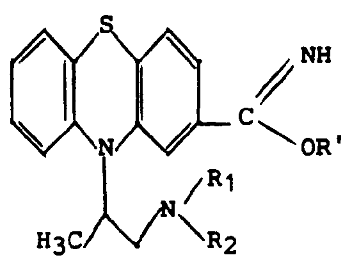

dans laquelle $R_1$ et $R_2$ sont définis comme ci-dessus, et R' est un radical alcoyle contenant 1 à 10 atomes de carbone, puis le cas échéant, pour obtenir un produit de formule générale (I), pour lequel R représente un radical $-\overset{+}{N}NR_1R_2R_3$, on fait réagir un produit de formule générale :

$$R_3 - X$$

dans laquelle $R_3$ est défini comme ci-dessus et X représente un atome d'halogène ou un radical alcoylsulfonyloxy ou phénylsulfonyloxy dont le radical phényle est éventuellement substitué par un ou plusieurs atomes d'halogène ou radicaux alcoyle ou nitro, puis transforme éventuellement le produit obtenu en un sel d'addition avec un acide.

**Patentansprüche**

**Patentansprüche für folgende Vertragsstaaten : AT, BE, CH, DE, FR, GB IT, LI, LU, NL, SE**

1. Phenothiazinderivat der allgemeinen Formel

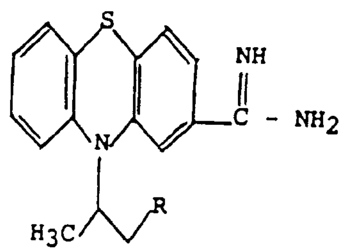

worin R bedeutet

- entweder einen Rest der allgemeinen Formel

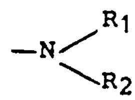

worin die Symbole $R_1$ und $R_2$, identisch oder voneinander verschieden, Alkyl-, Hydroxyalkyl-, Acetyloxyalkylreste bedeuten oder gemeinsam mit dem Stickstoffatom, an das sie gebunden sind, einen Heterocyclus mit 4 bis 7 Kettengliedern, welcher gegebenenfalls durch 1 oder 2 Alkyl-, Hydroxyalkyl- oder Acetyloxyalkylreste substituiert ist, bilden

- oder einen Rest der allgemeinen Formel

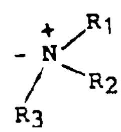

worin $R_1$ und $R_2$ wie vorstehend definiert sind, und $R_3$ einen Alkyl- oder Phenylalkylrest bedeutet, mit der Maßgabe, daß die Alkylreste und -teile 1 bis 4 Kohlenstoffatome in gerader oder verzweigter Kette enthalten, in seinen isomeren Formen oder deren Gemischen sowie ihre Additionssalze mit Säuren.

2. Phenothiazinderivat gemäß Anspruch 1, dadurch gekennzeichnet, daß das Symbol R entweder einen Rest $-NR_1R_2$ bedeutet, worin $R_1$ und $R_2$, identisch oder voneinander verschieden, Alkylreste mit 1 oder 2 Kohlenstoffatomen darstellen oder gemeinsam mit dem Stickstoffatom, an das sie gebunden sind, einen Heterocyclus mit 5 oder 6 Kettengliedern, gegebenenfalls substituiert durch 1 oder 2 Methylreste, bilden, oder R einen Rest $-\overset{+}{N}R_1R_2R_3$ bedeutet, worin $R_1$ und $R_2$ wie vorstehend definiert sind, und $R_3$ einen Alkylrest mit 1 oder 2 Kohlenstoffatomen bedeutet, in seinen isomeren Formen oder deren Gemischen sowie ihre Additionssalze mit Säuren.

3. Phenothiazinderivat gemäß Anspruch 1, worin R wie in Anspruch 2 definiert ist, in Form eines Isomerengemisches oder in der D-Form sowie ihre Additionsalze mit Säuren.

4. 10-[1-(Pyrolidin-1-yl)-prop-2-yl]-phenothiazin-2-carboxamidin in seinen isomeren Formen und deren Gemischen sowie deren Additionssalze mit Säuren.

**5.** 10-[1-(2-Methylpyrolidin-1-yl)-prop-2-yl]phenothiazin-2-carboxamidin in seinen isomeren Formen und deren Gemischen sowie deren Additionssalze mit Säuren.

**6.** 10-[1-(3,3-Dimethylpiperid-1-yl)-prop-2-yl]phenothiazin-2-carboxamidin in seinen isomeren Formen und deren Gemischen sowie deren Additionssalze mit Säuren.

**7.** 10-[1-(1-Methyl-1-pyrolidinio)-prop-2-yl]phenothiazin-2-carboxamidin in seinen isomeren Formen und deren Gemischen sowie deren Additionssalze mit Säuren.

**8.** Verfahren zur Herstellung eines Phenothiazinderivats gemäß Anspruch 1, dadurch gekennzeichnet, daß man Ammoniak mit einem Iminoether der allgemeinen Formel

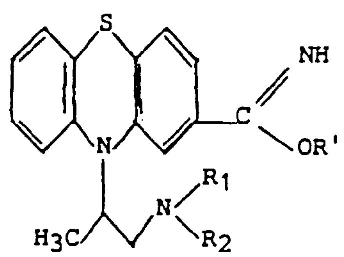

worin $R_1$ und $R_2$ wie in Anspruch 1 definiert sind, und R' einen Alkylrest mit 1 bis 10 Kohlenstoffatomen bedeutet, umsetzt, hiernach gegebenenfalls zur Erzielung eines Produkts gemäß Anspruch 1, worin R einen Rest $-\overset{+}{N}R_1R_2R_3$ bedeutet, mit einem Produkt der allgemeinen Formel

$$R_3 - X$$

umsetzt, worin $R_3$ wie in Anspruch 1 definiert ist, und X ein Halogenatom oder einen Alkylsulfonyloxy- oder Phenylsulfonyloxyrest, dessen Phenylrest gegebenenfalls durch ein oder mehrere Halogenatome oder Alkyl- oder Nitroreste substituiert ist, bedeutet, wonach man gegebenenfalls das erhaltene Produkt in ein Additionssalz mit einer Säure überführt.

**9.** Pharmazeutische Zusammensetzung, dadurch gekennzeichnet, daß sie zumindest ein Phenothiazinderivat gemäß Anspruch 1 in reiner Form oder in Form einer Assoziation mit jedem anderen verträglichen und pharmazeutisch annehmbaren Verdünnungsmittel oder Adjuvans enthält.

**Patentansprüch für folgende Vertragsstaaten : ES, GR**

**1.** Verfähren zur Herstellung eines Phenothiazinderivats der allgemeinen Formel

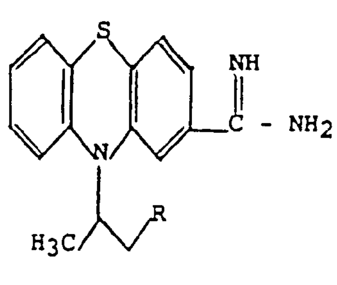

(I)

worin R bedeutet

- entweder einen Rest der allgemeinen Formel

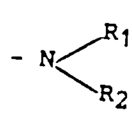

worin die Symbole $R_1$ und $R_2$, identisch oder voneinander verschieden, Alkyl-, Hydroxyalkyl-, Acetyloxyalkylreste bedeuten oder gemeinsam mit dem Stickstoffatom, an das sie gebunden sind, einen Heterocyclus mit 4 bis 7 Kettengliedern, der gegebenenfalls durch 1 oder 2 Alkyl-, Hydroxyalkyl- oder Acetyloxyalkylreste substituiert ist, bilden,

- oder einen Rest der allgemeinen Formel

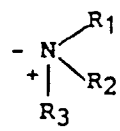

worin $R_1$ und $R_2$ wie vorstehend definiert sind, und $R_3$ einen Alkyl- oder Phenylalkylrest bedeutet, mit der Maßgabe, daß die Alkylreste und -teile 1 bis 4 Kohlenstoffatome in gerader oder verzweigter Kette enthalten, in seinen isomeren Formen oder deren Gemischen sowie von deren Additionssalzen mit Säuren, dadurch gekennzeichnet, daß man Ammoniak mit einem Iminoether der allgemeinen Formel

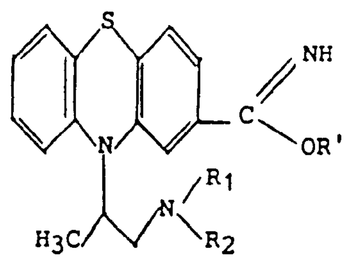

worin $R_1$ und $R_2$ wie vorstehend definiert sind, und R' einen Alkylrest mit 1 bis 10 Kohlenstoffatomen bedeutet, umsetzt, hiernach gegebenenfalls zur Erzielung eines Produkts der allgemeinen Formel (I), worin R für einen Rest -$\overset{+}{N}R_1R_2R_3$ steht, mit einem Produkt der allgemeinen Formel

$$R_3 - X$$

umsetzt, worin $R_3$ wie vorstehend definiert ist, und X ein Halogenatom oder einen Alkylsulfonyloxy- oder Phenylsulfonyloxyrest, dessen Phenylteil gegebenenfalls durch ein oder mehrere Halogenatome oder Alkyl- oder Nitroreste substituiert ist, bedeutet, und danach gegebenenfalls das erhaltene Produkt in ein Additionssalz mit einer Säure überführt.

## Claims

### Claims for the following Contracting States : AT, BE, CH, DE, FR, GB, IT, LI, LU, NL, SE

1. A phenothiazine derivative of general formula:

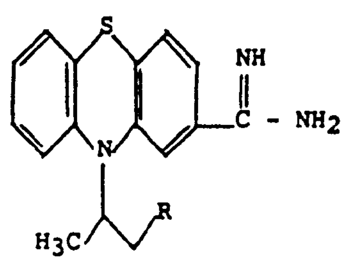

in which R represents

- either a radical of general formula:

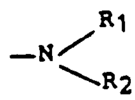

in which the symbols $R_1$ and $R_2$, which may be identical or different, represent alkyl, hydroxy alkyl or acetyloxyalkyl radicals or, together with the nitrogen atom to which they are attached, form a 4- to 7-membered heterocycle optionally substituted with 1 or 2 alkyl, hydroxyalkyl or acetyloxyalkyl radicals,

- or a radical of general formula:

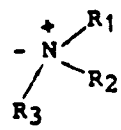

in which $R_1$ and $R_2$ are defined as above and $R_3$, represents an alkyl or phenylalkyl radical, on the understanding that the alkyl radicals and portions contain 1 to 4 carbon atoms in an unbranched or branched chain, in its isomeric forms or mixtures thereof, as well as its addition salts with acids.

2. A phenothiazine derivative according to Claim 1, characterised in that the symbol R represents either a radical $-NR_1R_2$ for which $R_1$ and $R_2$, which may be identical or different, represent alkyl radicals containing 1 or 2 carbon atoms or, together with the nitrogen atom to which they are attached, form a 5- or 6-membered heterocycle optionally substituted with 1 or 2 methyl radicals, or R represents a radical $-\overset{+}{N}R_1R_2R_3$, for which $R_1$ and $R_2$ are defined as above and $R_3$ represents an alkyl radical containing 1 or 2 carbon atoms, in its isomeric forms or mixtures thereof, as well as its addition salts with acids.

3. A phenothiazine derivative according to Claim 1 for which R is defined as in Claim 2, in the form of a mixture of isomers or in D form, as well as its addition salts with acids.

4. 10-[1-(1-Pyrrolidinyl)-2-propyl)-2-phenothiazine-carboxamidine, in its isomeric forms and mixtures thereof, as well as its addition salts with acids.

5. 10-[1-(2-Methyl-1-pyrrolidinyl)-2-propyl)-2-phenothiazinecarboxamidine, in its isomeric forms and mixtures thereof, as well as its addition salts with acids.

6. 10-[1-(3,3-Dimethyl-1-piperidyl)-2-propyl)-2-phenothiazinecarboxamidine, in its isomeric forms and mixtures thereof, as well as its addition salts with acids.

7. 10-[1-(1-Methyl-1-pyrrolidinio)-2-propyl)-2-phenothiazinecarboxamidine iodide, in its isomeric forms and mixtures thereof, as well as its addition salts with acids.

8. Process for preparing a phenothiazine derivative according to Claim 1, characterised in that ammonia is reacted with an imino ether of general formula:

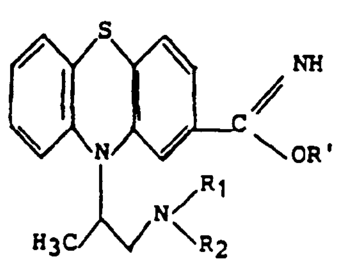

in which $R_1$ and $R_2$ are defined as in Claim 1 and R' is an alkyl radical containing 1 to 10 carbon atoms, and then, where appropriate, in order to obtain a product according to Claim 1 for which R represents a

radical $-\overset{+}{N}R_1R_2R_3$, a product of general formula:

$$R_3 - X$$

in which $R_3$ is defined as in Claim 1 and X represents a halogen atom, an alkylsulphonyloxy radical or a phenylsulphonyloxy radical in which the phenyl radical is optionally substituted with one or more halogen atoms or alkyl or nitro radicals, is reacted, and the product obtained is then optionally converted to an addition salt with an acid.

9. Pharmaceutical composition, characterised in that it contains at least one phenothiazine derivative according to Claim 1, in the pure state or in the form of a combination with any other compatible and pharmaceutically acceptable diluent or adjuvant.

**Claim for the following Contracting States : ES, GR**

1. Process for preparing a phenothiazine derivative of general formula:

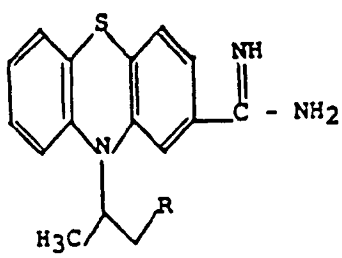

(I)

in which R represents

- either a radical of general formula:

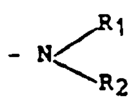

in which the symbols $R_1$ and $R_2$, which may be identical or different, represent alkyl, hydroxyalkyl or acetyloxyalkyl radicals or, together with the nitrogen atom to which they are attached, form a 4- to 7-membered heterocycle optionally substituted with 1 or 2 alkyl, hydroxyalkyl or acetyloxyalkyl radicals,

- or a radical of general formula:

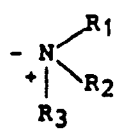

in which $R_1$ and $R_2$ are defined as above and $R_3$ represents an alkyl or phenylalkyl radical, on the understanding that the alkyl radicals and portions contain 1 to 4 carbon atoms in an unbranched or branched chain, in its isomeric forms or mixtures thereof, as well as its addition salts with acids, characterised in that ammonia is reacted with an imino ether of general formula:

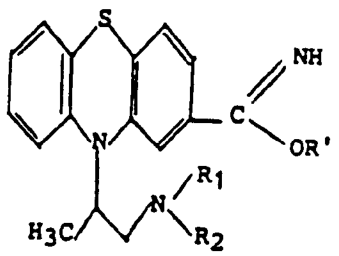

in which $R_1$ and $R_2$ are defined as above and R' is an alkyl radical containing 1 to 10 carbon atoms, and then, where appropriate, in order to obtain a product of general formula (I) for which R represents a radical $-\overset{+}{N}R_1R_2R_3$, a product of general formula:

$$R_3 - X$$

in which $R_3$ is defined as above and X represents a halogen atom, an alkylsulphonyloxy radical or a phenylsulphonyloxy radical in which the phenyl radical is optionally substituted with one or more halogen atoms or alkyl or nitro radicals, is reacted, and the product obtained is then optionally converted to an addition salt with an acid.